# EUROPEAN PATENT SPECIFICATION

(11) **EP 3 843 797 B1**
(45) Date of publication and mention of the grant of the patent: **12.02.2025**
(21) Application number: 19759722.2
(22) Date of filing: 27.08.2019
(51) Int. Cl.: A61L 24/00, A61L 24/02, A61L 27/12

(54) **SOLID SUSPENSION**
FESTE SUSPENSION
SUSPENSION SOLIDE

(30) Priority: 28.08.2018 GB 201813928
(43) Date of publication of application: 07.07.2021
(73) Proprietor: The University of Sheffield, Sheffield S10 2TN (GB)
(72) Inventor: HARRISON, Caroline Joanne, Sheffield S6 5BR (GB); HATTON, Paul Vincent, Sheffield S10 1EX (GB); HUTCHINSON, Rebecca Louise, Sheffield S7 2QX (GB); MILLER, Cheryl Ann, Sheffield (GB)
(74) Representative: Thurston, Joanna
(86) International application number: PCT/GB2019/052385
(87) International publication number: WO 2020/044028

(56) References cited:
- CN-A- 101 695 584
- US-A1- 2008 208 340
- US-A1- 2010 278 902

## Description

The invention relates to a solid suspension for use in bone regeneration and/or the repair of bone defects, in particular to a solid suspension comprising a source of at least one group II metal cation and a source of zinc cations, wherein the source of zinc cations comprises zinc oxide; and wherein the solid suspension is suitable for injection. The invention further relates to a bone graft, and to a method of preparing the suspension.

The promotion of bone regeneration (osteogenesis) remains a significant challenge in orthopaedic and dental surgery. The relatively recent commercial manufacture of solid suspensions of hydroxyapatite has provided surgeons with an injectable biomaterial that promotes bone tissue regeneration. WO 98/18719 describes a hydroxyapatite paste with excellent homogeneity, and US 7,387,785 describes nano-sized crystalline hydroxyapatite synthesis. A solid solution for use in bone tissue regeneration is described in WO 2017/137005, which discloses a bone regeneration solid solution with an optimised rate of calcium release. The solid solution is intended for use in the treatment of osteoporosis and comprises a combination of cations, including calcium, magnesium, strontium, barium and/or zinc. US 2010/278902 discloses a material, characterized in that it results from a sol-gel process and that its composition is characterized by the presence of the following elements in the proportions stated: SiO₂ : from 40 to 75%, CaO: from 15 to 30%, SrO: from 0.1 to 10%, P₂O₅ : from 0 to 10%, Na₂O: from 0 to 20%, MgO: from 0 to 10%, ZnO: from 0 to 10%, CaF₂ : from 0 to 5%, B₂O₃ : from 0 to 10%, Ag₂O: from 0 to 10%, Al₂O₃ : from 0 to 3%, MnO: from 0 to 10%, Others: from 0 to 10%, the percentages being percentages by weight relative to the total weight of the material. The composition is used for bone regeneration. CN101695584 describes an injectable bone repair material comprising hydroxyapatite doped with strontium ions and comprising zinc oxide.

It is desirable for these materials to have antimicrobial properties, ensuring that the solid suspension assists healing by preventing post-surgical infection. Silver-doped hydroxyapatite pastes have been found to offer one solution to this problem (Wilcock CJ, Stafford GP, Miller CA, Ryabenkova Y, Fatima M, Gentile P, Möbus G & Hatton PV (2017) Preparation and Antibacterial Properties of Silver-Doped Nanoscale Hydroxyapatite Pastes for Bone Repair and Augmentation. Journal of Biomedical Nanotechnology, 13(9), 1168-1176).

However, it would be desirable to offer alternative materials with bioregenerative and antimicrobial properties. The invention is intended to overcome or ameliorate at least some aspects of this problem.

Accordingly, in a first aspect of the invention there is provided a solid suspension for use in bone regeneration, and/or the repair of bone defects, comprising a source of strontium cations and a source of zinc cations, wherein the source of zinc cations comprises zinc oxide; and wherein the solid suspension is suitable for injection. The solid suspension ("paste") described herein has been found to have excellent antimicrobial properties. Without being bound by theory, this is believed to be as a result of the presence of both zinc and group II metal cations. It will often be the case that the group II metal cations are selected from calcium, strontium, magnesium and combinations thereof. Very often the group II metal cations will include, strontium or a mixture of strontium and calcium. The presence of strontium has been found to be particularly beneficial in enhancing the antimicrobial properties of the suspension.

As used herein the term "solid suspension" is intended to mean a homogenous mixture or suspension of a solid in a liquid, such as a sol or paste. Whether the solid suspension is a sol or a paste is dependent upon the relative amount of liquid and solid in the suspension.

The group II cations may be provided as sulfates, phosphates, carbonates, oxides, silicates, peroxides, sulfides and/or halides. Often they will be provided as phosphates, often hydroxyapatites. It is generally the case that where more than one group II cation is present, the sources of these are independent, such that each cation will be provided from a different source (although the counter-ion for each source will sometimes be the same such that calcium phosphate and strontium phosphate may be independently provided). Although dicationic compounds may be used (such as ion-exchanged dicationic calcium-strontium hydroxyapatite), generally if a combination of calcium and strontium is required, this will be provided as a calcium compound and a separate strontium compound.

Where present, the calcium cations in the solid suspension may be provided as a calcium compound selected from calcium sulfate anhydrite (CaSO₄), calcium sulfate hemihydrate (CaSO₄· 0.5H₂O), calcium sulfate dihydrate (CaSO₄· 2H₂O), monocalcium phosphate (Ca(H₂PO₄)₂), anhydrous dicalcium phosphate (CaHPO₄), tricalcium phosphate (Ca₃(PO₄)₂), tetracalcium phosphate (Ca₄(PO₄)₂O), octacalcium phosphate (Ca₈H₂(PO₄)₆· 5H₂O), hydroxyapatite (Ca₃(PO₄)₃(OH)), calcium carbonate (CaCO₃), calcium oxide (CaO) and calcium silicate (CaSiO₃) or combinations thereof. These compounds have been found to offer good biocompatibility and release of calcium cations during bone regeneration. Often the calcium compound comprise a phosphate, which may be selected from monocalcium phosphate, anhydrous dicalcium phosphate, tricalcium phosphate, tetracalcium phosphate, octacalcium phosphate, hydroxyapatite or combinations thereof, most often the calcium compound will comprise hydroxyapatite, often the calcium compound will consist of or consist essentially of hydroxyapatite. This is for biocompatibility reasons. As such, the solid suspension may comprise a calcium compound which may be hydroxyapatite.

In the solid suspension the group II cation compound, such as the calcium compound, may be present as a nano-compound. As used herein the term "nano" is intended to denote particles of a mean particle diameter (widest point) in the range 1 nm - 100 nm, often 5 nm - 75 nm, 25 nm - 50 nm or 50 nm - 100 nm. Within these ranges the nano-compound, often nano-hydroxyapatite, closely resembles the scale found in natural human bone.

The strontium cations are provided as a strontium compound selected from strontium sulfate (SrSO₄), strontium phosphate (Sr₃(PO₄)₂), strontium carbonate (SrCO₃), strontium oxide (SrO), strontium peroxide (SrO₂), strontium phosphide (Sr₃P₂), strontium sulfide (SrS), strontium chloride (SrCl₂), strontium-substituted hydroxyapatite (Sr₅(PO₄)₃(OH)) and strontium ranelate (C₁₂H₆N₂O₈SSr₂) or combinations thereof. Often, the strontium compound comprises strontium-substituted hydroxyapatite. As such, the solid suspension may comprise a strontium compound which may be strontium-substituted hydroxyapatite.

The ratio of calcium:strontium cations in the suspension is in the range 0:100 - 99:1, or 1:99 - 70:30. As such, where the ratio is 0:100 calcium:strontium, the calcium cations may be absent. The presence of strontium has been found to enhance the antimicrobial and osteogenic properties of the solid suspension. Accordingly, a source of group II cations is strontium, often strontium-substituted hydroxyapatite, often the group II cations will be entirely supplied by the provision of strontium-substituted hydroxyapatite (100% strontium-substituted hydroxyapatite or a ratio of calcium:strontium of 0:100). Alternatively, there may be as little as 1% strontium, which may be provided as strontium-substituted hydroxyapatite, the remaining group II cation optionally being provided in the form of a group II cation substituted-hydroxyapatite. Often this will comprise calcium hydroxyapatite. Often the ratio of calcium:strontium is greater than or equal to 70:30 (30% or more strontium). Even where the group II cation is only 30% strontium, the suspensions have been found to offer excellent antimicrobial and osteogenic properties.

The solid suspension will comprise zinc oxide, often the zinc oxide will be of mean particle size in the range 0.01 µm - 100 µm, often in the range 0.1 µm - 50 µm or 0.2 µm to 10 µm. At sizes in this range the zinc oxide has been found to mix well with the solid suspension forming good homogeneous pastes.

Often the solid suspension will comprise in the range 20 - 60 wt% solid, often 30 - 45 wt% solid, or 35 - 45 wt% solid; the remaining 40 - 80 wt% being a liquid component. Often the solid suspension will comprise in the range 55 - 70 wt% or 55 - 65 wt% liquid component. At these levels of solid, the solid suspension will be in the form of a paste, namely a semi-fluid composition. This ensures that the solid suspension is easily to manipulate, in particular being injectable to an implantation location, without being unduly fluid such that it will remain at the implantation location after application and without flowing away.

It may be the case that the liquid component comprises water, for its ready accessibility and biocompatibility, often deionised water.

The use of zinc oxide can be beneficial as zinc oxide can generate reactive oxygen species, which contribute to the antimicrobial effect of the suspension. Typically, the zinc oxide will be present in the range 0.25 wt% - 5.0 wt% of the solid suspension, often in the range 0.5 wt% to 3.0 wt%, often 1.0 wt% - 2.0 wt%. At these ranges good antimicrobial activity is observed.

The solid suspension for a use selected from bone regeneration and/or in the repair of bone defects; the treatment of bone defects arising from osteoporosis, wherein the solid suspension is to be administered to the bone defect site by injection; and/or the treatment of bone defects arising from osteoarthritis, wherein the solid suspension is to be administered to the bone defect site by injection.

The solid suspension may be at least partially bioresorbable, allowing for dissolution of any bone graft formed from the solid suspension as bone regrowth occurs. Often it will be the case that the solid suspension is for use in bone regeneration, often the solid suspension will be used in dental applications, or in orthopaedic or spinal bone regeneration.

In a second aspect of the invention there is provided a bone graft which may be bioresorbable, comprising the solid suspension of the first aspect of the invention.

In a third aspect of the invention there is provided a method of preparing a solid suspension for use in bone regeneration and/or the repair of bone defects, comprising: mixing a source of strontium cations with zinc oxide.

It may be that the group II cations are calcium and strontium cations, in such cases the source of calcium cations may be a calcium compound such as hydroxyapatite and the source of strontium ions may be a strontium compound such as strontium-substituted hydroxyapatite.

Also described but not claimed is a method of forming a bone graft comprising delivering a solid suspension of the first aspect of the invention to an implantation location. The bone graft may be bioresorbable. Often the implantation location is in the human or animal body, often the implantation location is orthopaedic, spinal or dental.

In a fifth aspect of the invention there is provided the solid suspension of the first aspect of the invention for use in bone regeneration and/or the repair of bone defects. Often the bone regeneration will be cosmetic, although solid suspensions for use in the treatment of conditions such as osteoporosis and osteoarthritis are also envisaged. This is particularly the case where the bone regeneration is dental.

There is therefore provided an at least partially resorbable solid suspension for use in bone regeneration comprising in the range 35 - 45 wt% solid, the solid comprising: calcium cations provided as a calcium compound comprising nano-hydroxyapatite, strontium cations provided as a strontium compound comprising strontium-substituted hydroxyapatite, and zinc cations, provided as zinc oxide of particle size in the range 0.2 µm to 10 µm wherein the zinc oxide may be present in the range 0.25 to 5.0 wt% of the solid suspension. A ratio of calcium:strontium cations may be in the range 0:100 - 70:30.

Unless otherwise stated, each of the integers described may be used in combination with any other integer as would be understood by the person skilled in the art. Further, although all aspects of the invention preferably "comprise" the features described in relation to that aspect, it is specifically envisaged that they may "consist" or "consist essentially" of those features outlined in the claims. In addition, all terms, unless specifically defined herein, are intended to be given their commonly understood meaning in the art.

Further, in the discussion of the invention, unless stated to the contrary, the disclosure of alternative values for the upper or lower limit of the permitted range of a parameter, is to be construed as an implied statement that each intermediate value of said parameter, lying between the smaller and greater of the alternatives, is itself also disclosed as a possible value for the parameter.

In order that the invention may be more readily understood, it will be described further with reference to the figures and to the specific examples hereinafter.
Figure 1 shows the particle size distribution of zinc oxide used in the test examples;
Figure 2 is a graph illustrating the biocompatibility of the pastes/suspensions tested. Calcium hydroxyapatite pastes containing 0, 1, 2 or 3 wt% zinc oxide, no paste and strontium-substituted hydroxyapatite paste were tested;
Figure 3 is a graph illustrating the antibacterial effect of increasing concentration of zinc oxide in a hydroxyapatite paste. Shown are the number of viable S. *aureus* after 24 h. Error bars ± S.E.M. Significance: * p<0.05;
Figure 4 is a graph illustrating the reduction in the number of viable S. *aureus* after 24 h at different concentrations of zinc oxide in hydroxyapatite, based on the data of Figure 3. Error bars ± S.E.M; and
Figure 5 is a graph illustrating the antibacterial effect of each of hydroxyapatite, hydroxyapatite + 2 wt% zinc oxide, strontium-substituted hydroxyapatite, and strontium-substituted hydroxyapatite + 2 wt% zinc oxide. Shown are the number of viable S. *aureus* attached to HA and SrHA pastes containing 2 wt% ZnO after 24 h. Error bars ± S.E.M. Significance: * p<0.05.

### Examples

### Paste manufacture

Pastes were prepared by mixing zinc oxide powder with hydroxyapatite (HA) slurries. Depending on the example, the hydroxyapatite slurries may be calcium, strontium or combinations thereof in the cationic ratios discussed. A portion of the water content was then evaporated in a drying oven resulting in a paste consistency. The amount of zinc oxide was calculated as a percentage of the final HA paste (HA solids + water) with the residual solid content of the HA paste being in the region of 38 - 40 wt%.

### Test Methods

### Antibacterial evaluation

The ability of the pastes to prevent bacterial colonisation on the paste surface was assessed using a biofilm initialisation model. The pastes were tested for antibacterial activity against clinical isolates of Staphylococcus aureus (S235). The attachment and survival of bacteria to the surface of the pastes was assessed after a 24 h incubation.

10 mm (±1 mm) lengths of sterile pastes extruded from a standard luer lock syringe were placed into sterile 1 ml Eppendorfs, with 3 samples prepared for each paste. 500 µL of sterile PBS (phosphate buffered saline) was added to each Eppendorf. A bacterial suspension of OD 0.05 was prepared in PBS and 500 µL of bacterial suspension was added to the Eppendorf tubes containing paste (n=3). The tubes were incubated at 37 °C, 5 % CO₂ for 20 h. The PBS was removed from all tubes and the paste lengths were washed twice with 1 ml PBS. To assess the number of viable bacteria attached to the paste after the overnight incubation the pastes were suspended and serial dilutions were plated on agar plates to allow for colonies to be counted. In detail, the washed pastes were suspended in 1 ml PBS using a vortex. Two serial dilutions were then performed per sample in PBS and the following dilutions were plated up by placing 10 µL sample onto BHI agar plates: 1 in 10⁰, 10¹, 10², 10³ and 10⁴. The plates were incubated overnight at 37 °C, 5 % CO₂ and the colonies were counted at an appropriate dilution where single colonies could easily be identified.

Statistical analysis was carried out on the log numbers from the experiment using SPSS software. The statistical analysis firstly involved a one way ANOVA test. The post hoc test was then selected based on the homogeneity of the variance between the experimental groups. Levene's test was used to determine the homogeneity of the variances. If the variances were not significantly different from each other, Tukey's multiple comparisons test was used. If the variances were significantly different from each other, Games-Howell comparison was applied.

### Biocompatibility of pastes

The biocompatibility of the pastes was investigated by seeding 50,000 MG63 cells per well with 1 mL basal media (made up of the following v/v %: α-MEM supplemented with 10 % foetal calf serum, 1 % I-alanyl-I-glutamine, 1 % penicillin-streptomycin, 1 % non-essential amino acids) in a 24 well plate. The cells were incubated at 37 °C, 5 % CO₂ for 24 h after which the media was removed and 0.9 mL media was added. Permeable Millicell^{®} hanging inserts (0.4 µm pore size, Merck Millipore) were then placed in each well and 0.2 mL complete media was added inside each insert. 0.1 mL paste was then added to each insert in triplicate and the plates were incubated at 37 °C, 5 % CO₂ for an additional 24 h. After the incubation the inserts were removed and the cells were imaged using light microscopy. The media was removed from the cells and 0.5 mL of a 10 % (v/v) PrestoBlue^{®} solution in complete media was added to each well. PrestoBlue^{®} solution was also added in triplicate to empty wells as a control to subtract from the fluorescence values obtained. The samples were incubated at 37 °C, 5 % CO₂ until appropriate colour change was observed. At each time point 0.2 mL solution was placed into a 96 well plate. The fluorescence of the solutions was measured using a plate reader with an excitation wavelength of 535 nm and an emission wavelength of 590 nm. The same statistical approach was used as described above for the antibacterial testing.

### Test Results

### Particle Size Distribution of Zinc Oxide

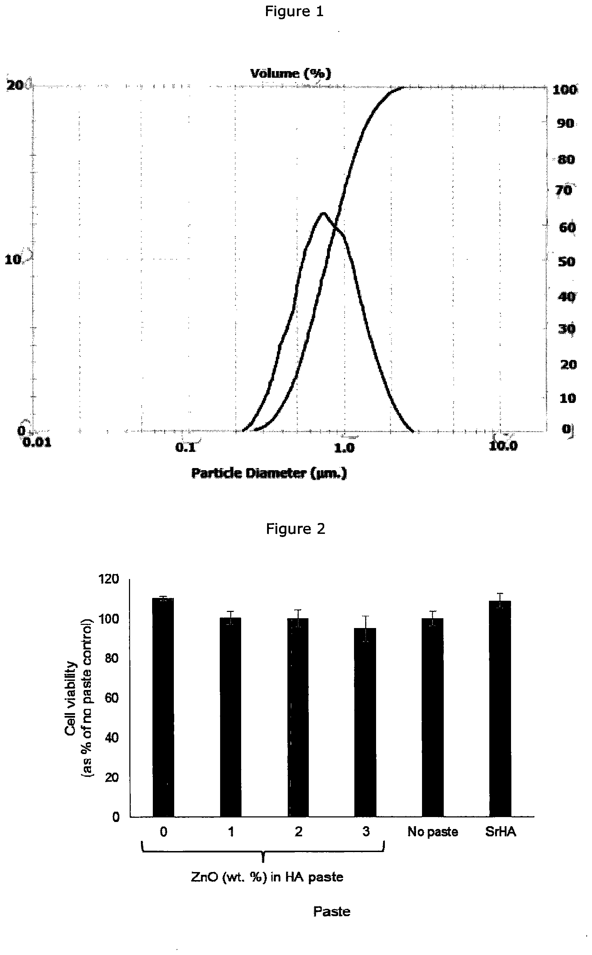

The particle size distribution of the zinc oxide used in these tests was as shown in Tables 1 and 2 below. Figure 1 illustrates this in graphical form.

**Table 1. Particle size distribution**

| **Under** | **0.4 µm** | **0.6 µm** | **0.8 µm** | **1 µm** | **3 µm** |
|---|---|---|---|---|---|
| % | 5 - 15 | 20 - 40 | 45 - 60 | 60 - 80 | 100 |

**Table 2. Particle size distribution**

| | **D10** | **D50** | **D90** |
|---|---|---|---|
| µm | 0.30 - 0.45 | 0.70 - 0.85 | 1.20 - 1.70 |

### Biocompatibility of Pastes

Figure 2 shows the excellent biocompatibility of the suspensions. Specifically it shows good cell viability (MG63 cells) with calcium hydroxyapatite pastes containing 0, 1, 2 and 3 wt% zinc oxide, with strontium-substituted hydroxyapatite paste and in the absence of paste. It is clear that cell viability is not significantly affected by the presence of the suspensions.

### Antibacterial Activity of Zinc Oxide

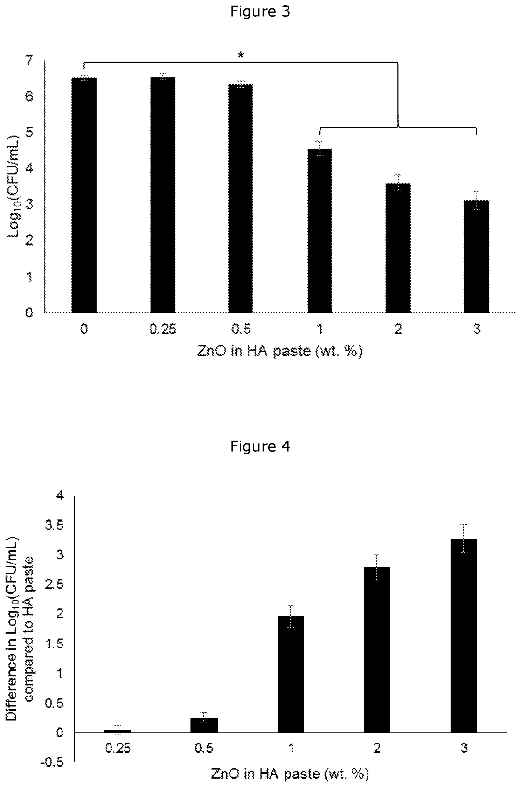

Figures 3 and 4 show the antibacterial activity of hydroxyapatite pastes containing zinc oxide in a range of concentrations. It can be seen that the presence of zinc oxide enhances the antibacterial properties of the hydroxyapatite, this enhancement becoming significant when the zinc oxide is present at a level above 0.25 wt% of the solid.

### Antibacterial Activity of Zinc Oxide combined with Strontium

The antibacterial activity of the following four compositions was measured and compared:
1. hydroxyapatite,
2. hydroxyapatite + 2 wt% of the solid suspension zinc oxide,
3. strontium-substituted hydroxyapatite, and
4. strontium-substituted hydroxyapatite + 2 wt% of the solid suspension zinc oxide

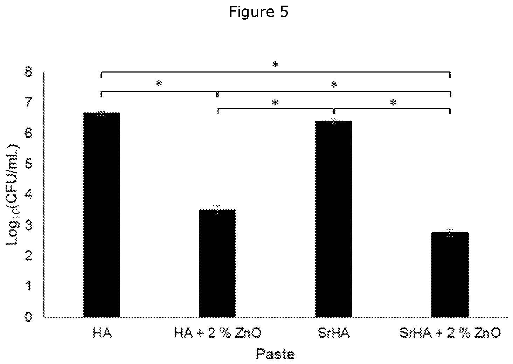

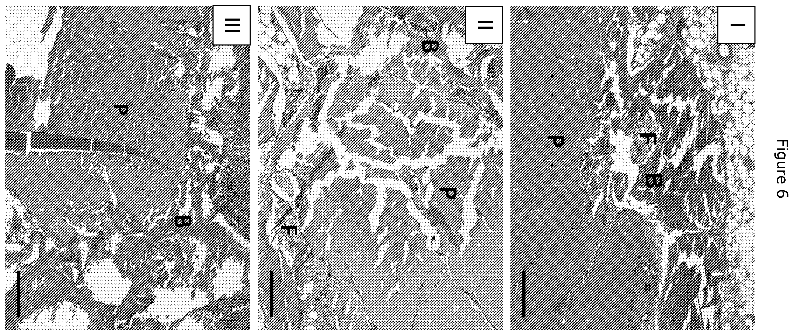

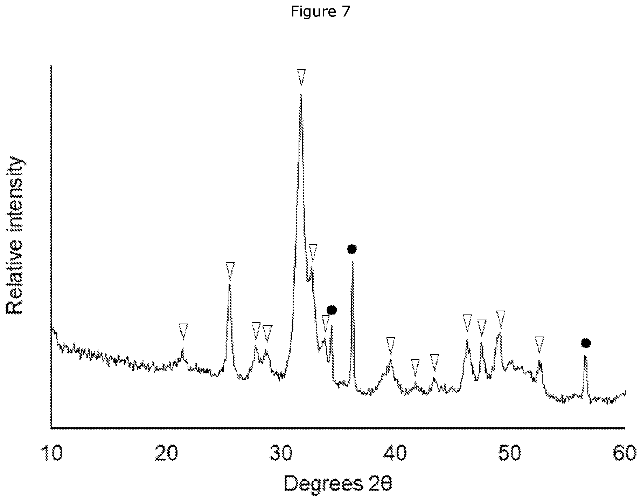

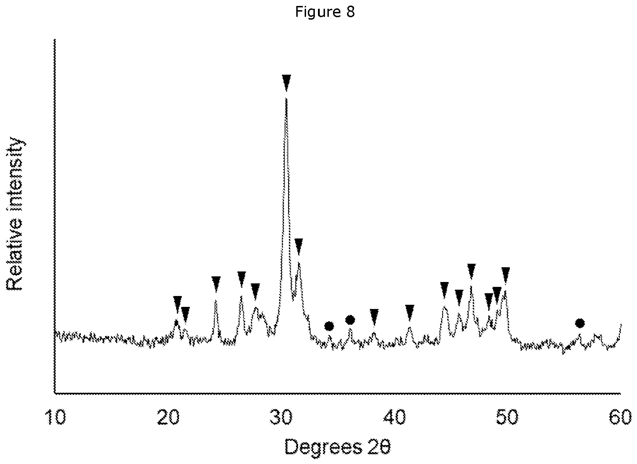

As can be seen from Figure 5, and discussed above, the addition of zinc oxide alone enhances the antibacterial properties of hydroxyapatite. Figure 5 also shows that the presence of strontium enhances the antibacterial properties of hydroxyapatite. Unexpectedly, however, where both zinc oxide and strontium are present a synergistic effect is observed. Specifically, it has been shown that the combination of the zinc and strontium has a greater antibacterial effect in terms of the reduction of bacterial numbers than would be expected from an additive effect of each of the zinc and strontium compounds alone (i.e. the number of viable bacteria attached to the SrHA + 2 % ZnO is significantly lower than the number of viable bacteria attached to SrHA and HA + 2 wt% ZnO pastes). Therefore, the zinc oxide and strontium appear to be working together, in synergy, to provide an antibacterial effect.

It would be appreciated that the methods of the invention are capable of being implemented in a variety of ways, only a few of which have been illustrated and described above.

## Claims

1. A solid suspension for use in bone regeneration and/or the repair of bone defects, comprising a source of strontium cations and a source of zinc cations, wherein the source of zinc cations comprises zinc oxide; and wherein the solid suspension is suitable for injection.

2. A solid suspension for use according to claim 1, further comprising group II metal cations selected from calcium, magnesium, or combinations thereof.

3. A solid suspension for use according to claim 2, wherein the calcium cations are provided as a calcium compound selected from calcium sulfate anhydrite (CaSO₄), calcium sulfate hemihydrate (CaSO₄· 0.5H₂O), calcium sulfate dihydrate (CaSO₄· 2H₂O), monocalcium phosphate (Ca(H₂PO₄)₂), dicalcium phosphate anhydrous (CaHPO₄), tricalcium phosphate (Ca₃(PO₄)₂), tetracalcium phosphate (Ca₄(PO₄)₂O), octacalcium phosphate (Ca₈H₂(PO₄)₆· 5H₂O), hydroxyapatite (Ca₃(PO₄)₃(OH)), calcium carbonate (CaCO₃), calcium oxide (CaO) and calcium silicate (CaSiO₃) or combinations thereof; optionally wherein the calcium compound comprises a phosphate; optionally wherein the calcium compound comprises hydroxyapatite.

4. A solid suspension for use according to any preceding claim, wherein the strontium cations are provided as a strontium compound selected from strontium sulfate (SrSO₄), strontium phosphate (Sr₃(PO₄)₂), strontium carbonate (SrCO₃), strontium oxide (SrO), strontium peroxide (SrO₂), strontium phosphide (Sr₃P₂), strontium sulfide (SrS), strontium chloride (SrCl₂), strontium-substituted hydroxyapatite (Sr₅(PO₄)₃(OH)) and strontium ranelate (C₁₂H₆N₂O₈SSr₂) or combinations thereof; optionally wherein the strontium compound comprises strontium-substituted hydroxyapatite.

5. A solid suspension for use according to claim 4, wherein the hydroxyapatite comprises nano-hydroxyapatite.

6. A solid suspension for use according to claim 4 or claim 5, comprising a ratio of calcium:strontium cations in the range 0:100 - 99:1.

7. A solid suspension for use according to any preceding claim, wherein the zinc oxide is of mean particle size in the range 0.01 µm - 100 µm.

8. A solid suspension for use according to any preceding claim, comprising in the range 20 - 60 wt% solid.

9. A solid suspension for use according to any preceding claim, comprising zinc oxide in the range 0.25 - 5.0 wt% of the solid suspension.

10. A solid suspension for use according to any preceding claim, wherein the solid suspension is at least partially bioresorbable.

11. A solid suspension for use according to any preceding claim, for a use selected from bone regeneration and/or in the repair of bone defects; the treatment of bone defects arising from osteoporosis, wherein the solid suspension is to be administered to the bone defect site by injection; and/or the treatment of bone defects arising from osteoarthritis, wherein the solid suspension is to be administered to the bone defect site by injection.

12. A solid suspension for use according to any preceding claim, for a use selected from orthopaedic or spinal bone regeneration, dental applications, and/or in the repair of bone defects arising from osteoporosis or osteoarthritis.

13. A bone graft, comprising the solid suspension for use of any preceding claim.

14. A method of preparing a solid suspension according to any of claims 1 to 12, comprising: mixing a source of strontium cations with zinc oxide.

15. A solid suspension for use according to any of claims 1 - 12, for cosmetic bone regeneration, optionally wherein the cosmetic bone regeneration is dental.

## Patentansprüche

1. Feste Suspension zur Verwendung bei der Knochenregeneration und/oder der Reparatur von Knochendefekten, umfassend eine Quelle für Strontium-Kationen und eine Quelle für Zink-Kationen, wobei die Quelle für Zink-Kationen Zinkoxid umfasst, und wobei die feste Suspension zur Injektion geeignet ist.

2. Feste Suspension zur Verwendung nach Anspruch 1, ferner umfassend Kationen von Metallen der Gruppe II, ausgewählt aus Calcium, Magnesium oder Kombinationen davon.

3. Feste Suspension zur Verwendung nach Anspruch 2, wobei die Calcium-Kationen als Calciumverbindung bereitgestellt sind, ausgewählt aus Calciumsulfatanhydrit (CaSO₄), Calciumsulfathemihydrat (CaSO₄·0,5H,0), Calciumsulfatdihydrat (CaSO₄·2H,0), Monocalciumphosphat (Ca(H₂PO₄)₂), wasserfreiem Dicalciumphosphat (CaHPO₄), Tricalciumphosphat (Ca₃(PO₄)₂), Tetracalciumphosphat (Ca₄(PO₄)₂O), Octacalciumphosphat (Ca₈H₂(PO₄)₆· 5H₂0), Hydroxylapatit (Ca₅(PO₄)₃(OH)), Calciumcarbonat (CaCO₃), Calciumoxid (CaO) und Calciumsilikat (CaSiO₃) oder Kombinationen davon, wobei die Calciumverbindung optional ein Phosphat umfasst, wobei die Calciumverbindung optional Hydroxylapatit umfasst.

4. Feste Suspension zur Verwendung nach einem der vorhergehenden Ansprüche, wobei die Strontium-Kationen als Strontiumverbindung bereitgestellt sind, ausgewählt aus Strontiumsulfat (SrSO₄), Strontiumphosphat (Sr₃(PO₄)₂), Strontiumcarbonat (SrCO₃), Strontiumoxid (SrO), Strontiumperoxid (SrO₂), Strontiumphosphid (Sr₃P₂), Strontiumsulfid (SrS), Strontiumchlorid (SrCl₂), mit Strontium substituiertes Hydroxylapatit (Sr₅(PO₄)₃(OH)) und Strontiumranelat (C₁₂H₆N₂O₈SSr₂) oder Kombinationen davon, wobei die Strontiumverbindung optional mit Strontium substituiertes Hydroxylapatit umfasst.

5. Feste Suspension zur Verwendung nach Anspruch 4, wobei das Hydroxylapatit Nano-Hydroxylapatit umfasst.

6. Feste Suspension zur Verwendung nach Anspruch 4 oder Anspruch 5, umfassend ein Verhältnis von Calcium- zu Strontium-Kationen im Bereich von 0:100 bis 99:1.

7. Feste Suspension zur Verwendung nach einem der vorhergehenden Ansprüche, wobei das Zinkoxid eine mittlere Partikelgröße im Bereich von 0,01 µm bis 100 µm aufweist.

8. Feste Suspension zur Verwendung nach einem der vorhergehenden Ansprüche, umfassend Feststoffe im Bereich von 20 bis 60 Gewichtsprozent.

9. Feste Suspension zur Verwendung nach einem der vorhergehenden Ansprüche, umfassend Zinkoxid im Bereich von 0,25 bis 5,0 Gewichtsprozent der festen Suspension.

10. Feste Suspension zur Verwendung nach einem der vorhergehenden Ansprüche, wobei die feste Suspension zumindest teilweise bioresorbierbar ist.

11. Feste Suspension zur Verwendung nach einem der vorhergehenden Ansprüche, für eine Verwendung ausgewählt aus Knochenregeneration und/oder bei der Reparatur von Knochendefekten, der Behandlung von Knochendefekten, die durch Osteoporose entstehen, wobei die feste Suspension durch Injektion an die Knochendefektstelle verabreicht werden soll, und/oder der Behandlung von Knochendefekten, die durch Osteoarthritis entstehen, wobei die feste Suspension durch Injektion an die Knochendefektstelle verabreicht werden soll.

12. Feste Suspension zur Verwendung nach einem der vorhergehenden Ansprüche, für eine Verwendung ausgewählt aus orthopädischer oder spinaler Knochenregeneration, zahnmedizinischen Anwendungen und/oder bei der Reparatur von Knochendefekten, die durch Osteoporose oder Osteoarthritis entstehen.

13. Knochentransplantat, umfassend die feste Suspension zur Verwendung nach einem der vorhergehenden Ansprüche.

14. Verfahren zur Herstellung einer festen Suspension nach einem der Ansprüche 1 bis 12, umfassend: Mischen einer Quelle von Strontium-Kationen mit Zinkoxid.

15. Feste Suspension zur Verwendung nach einem der Ansprüche 1 bis 12 zur kosmetischen Knochenregeneration, wobei die kosmetische Knochenregeneration optional dental ist.

## Revendications

1. Une suspension solide destinée à être utilisée dans la régénération osseuse et/ou la réparation de défauts osseux, comprenant une source de cations de strontium et une source de cations de zinc, la source de cations de zinc comprenant de l'oxyde de zinc et la suspension solide étant approprié pour être injectée.

2. Une suspension solide destinée à être utilisée selon la revendication 1, comprenant en outre des cations métalliques du groupe II sélectionnés parmi le calcium, le magnésium ou des combinaisons de ceux-ci.

3. Une suspension solide destinée à être utilisée selon la revendication 2, dans laquelle les cations de calcium sont amenés sous forme d'un composé de calcium sélectionné parmi le sulfate de calcium naturel de calcium anhydre (CaSO₄), le sulfate de calcium hémi-hydraté (CaSO₄.0,5H₂O), le sulfate de calcium dihydraté (CaSO₄.2H₂O), le phosphate monocalcique [Ca(H₂PO₄)₂], le phosphate dicalcique anhydre (CaHPO₄), le phosphate tricalcique [Ca₃(PO₄)₂] le phosphate tétracalcique [Ca₄(PO₄)₂O], le phosphate octocalcique [Ca₈H₂(PO₄)₆.5H₂O], l'hydroxyapatite (Ca₅(PO₄)₃)(OH)), le carbonate de calcium (CaCO₃), l'oxyde de calcium (CaO) et le silicate de calcium (CaSiO₃) ou des combinaisons de ceux-ci ; éventuellement dans laquelle le composé de calcium comprend un phosphate ; éventuellement dans laquelle le composé de calcium comprend de l'hydroxyapatite.

4. Une suspension solide destinée à être utilisée selon l'une quelconque des revendications précédentes, dans laquelle les cations de strontium sont amenés sous forme d'un composé de strontium sélectionné parmi le sulfate de strontium (SrSO₄), le phosphate de strontium (Sr₃(PO₄)₂), le carbonate de strontium (SrCO₃), l'oxyde de strontium (SrO), le peroxyde de strontium (SrO₂), le phosphure de strontium (Sr₃P₂), le sulfure de strontium (SrS), le chlorure de strontium (SrCl₂), l'hydroxyapatite substituée par du strontium [Sr₅(PO₄)₃(OH)] et le ranélate de strontium (C₁₂H₆N₂O₈SSr₂) ou des combinaisons de ceux-ci ; éventuellement dans laquelle le composé de strontium comprend de l'hydroxyapatite substituée par du strontium.

5. Une suspension solide destinée à être utilisée selon la revendication 4, dans laquelle l'hydroxyapatite comprend de la nano-hydroxyapatite.

6. Une suspension solide destinée à être utilisée selon la revendication 4 ou la revendication 5, présentant un rapport cations de calcium/cations de strontium dans la plage de 0/100 à 99/1.

7. Une suspension solide destinée à être utilisée selon l'une quelconque des revendications précédentes, dans laquelle l'oxyde de zinc présente une dimension de particules moyenne dans la plage de 0,01 µm à 100 µm.

8. Une suspension solide destinée à être utilisée selon l'une quelconque des revendications précédentes, présentant une proportion de solides dans la plage de 20 à 60 % en poids.

9. Une suspension solide destinée à être utilisée selon l'une quelconque des revendications précédentes, présentant une proportion d'oxyde de zinc dans la plage de 0,25 à 5,0 % en poids du poids de la suspension solide.

10. Une suspension solide destinée à être utilisée selon l'une quelconque des revendications précédentes, dans laquelle la suspension solide est au moins partiellement biorésorbable.

11. Une suspension solide destinée à être utilisée selon l'une quelconque des revendications précédentes, pour une utilisation sélectionnée parmi la régénération osseuse et/ou la réparation de défauts osseux ; le traitement de défauts osseux résultant de l'ostéoporose, auquel cas la suspension solide devra être administrée par injection au site du défaut osseux; et/ou le traitement de défauts osseux résultant de l'arthrose, auquel cas la suspension solide devra être administrée par injection au site du défaut osseux.

12. Une suspension solide destinée à être utilisée selon l'une quelconque des revendications précédentes, pour une utilisation sélectionnée parmi la régénération osseuse orthopédique ou rachidienne, les applications dentaires /ou la réparation de défauts osseux résultant de l'ostéoporose ou de l'arthrose.

13. Une greffe osseuse, comprenant la suspension solide destinée à être utilisée selon l'une quelconque des revendications précédentes.

14. Un procédé de préparation d'une suspension solide selon l'une quelconque des revendications 1 à 12, comprenant le mélange d'une source de cations de strontium avec de l'oxyde de zinc.

15. Une suspension solide destinée à être utilisée selon l'une quelconque des revendications 1 à 12, pour la régénération osseuse cosmétique, éventuellement dans laquelle la régénération osseuse cosmétique est dentaire.
